(19) Europäisches Patentamt
European Patent Office
Office européen des brevets

(11) Publication number: **0 305 954**
**A1**

(12) # EUROPEAN PATENT APPLICATION

(21) Application number: 88114076.8

(51) Int. Cl.⁴: **A61K 6/08**

(22) Date of filing: 29.08.88

(30) Priority: 28.08.87 JP 215936/87

(43) Date of publication of application:
08.03.89 Bulletin 89/10

(84) Designated Contracting States:
DE FR GB

(71) Applicant: DAIKIN INDUSTRIES LIMITED
Umeda Center Building 4-12, Nakazaki-nishi
2-chome Kita-ku
Osaka-shi Osaka-fu(JP)

(72) Inventor: Yamazaki, Noboru
11-7, Seta 2-chome Setagaya-ku
Tokyo-to(JP)
Inventor: Kurata, Shigeaki
1-5, Izumi-cho 1-chome
Kokubunji-shi Tokyo-to(JP)
Inventor: Yagi, Toshiharu
4-23, Hanayashiki soen 1-chome
Takarazuka-shi Hyogo-ken(JP)
Inventor: Inukai, Hiroshi
11-710, Showaen 8-ban
Settsu-shi Osaka-fu(JP)

(74) Representative: Barz, Peter, Dr. et al
Patentanwälte Dipl.-Ing. G. Dannenberg Dr.
P. Weinhold, Dr. D. Gudel Dipl.-Ing. S.
Schubert, Dr. P. Barz Siegfriedstrasse 8
D-8000 München 40(DE)

(54) **Dental material.**

(57) A dental material comprising a copolymer which contains at least 10% by weight of at least one structural unit derived from the following monomers

EP 0 305 954 A1

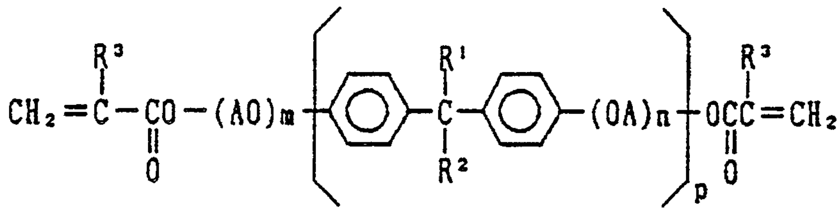 (1)

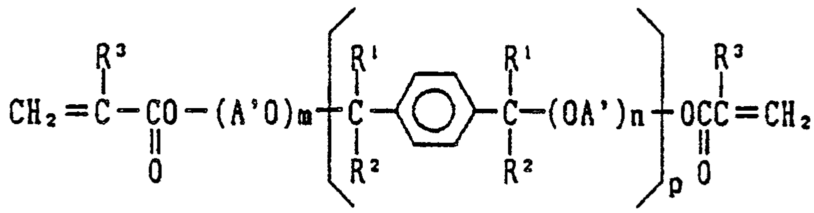 (2)

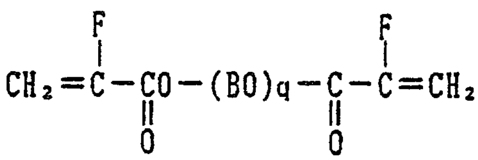 (3)

wherein $R^1$ and $R^2$ are each H, $CH_3$ or $CF_3$, $R^3$ is H, $CH_3$ or F, A is $-CH_2-$, $-CH_2CH_2-$, $-CH_2CH(CH_3)-$, $-CH(OH)-$, $-CH_2CH(OH)CH_2-$, $-CH(OCOCH_3)-$ or $-CH_2CH(OCOCH_3)CH_2-$, A′ is the same group as A or $-CH_2CH_2CH_2-$, B is alkylene group having 2 to 10 carbon atoms, m and n are each zero or a number of 1 to 5, p is a number of 1 to 3, q is a number of 1 to 5, provided that at least one of $R^1$ and $R^2$ is $CF_3$ when $R^3$ is H or $CH_3$.

## Dental material

The present invention relates to a dental material comprising a copolymer which is obtained from a fluorine-containing acrylic ester monomer.

Recently with developments in polymer chemistries and industries, various dental materials are investigated which are prepared from a high-molecular-weight material.

Though attention is paid, for example, to a photopolymerized type restoration material, crown material or adhesive high-molecular-weight material, these functional materials are quite novel dental materials and are considered to promote technical innovation in the dental medical system.

As examples of these dental materials, polymers of the following compound are used.

3G : Triethylene glycol dimethacrylate

$CH_2 = C(CH_3)COO(CH_2CH_2O)_3\text{-}COC(CH_3) = CH_2$

Bis-GMA : 2,2-Bis[4-(3-methacryloxy-2-hydroxypropoxy)phenyl]propane

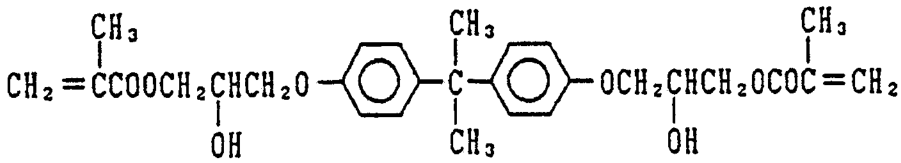

Bis-MEPP : 2,2-Bis(4-methacryloxyethoxyphenyl)propane

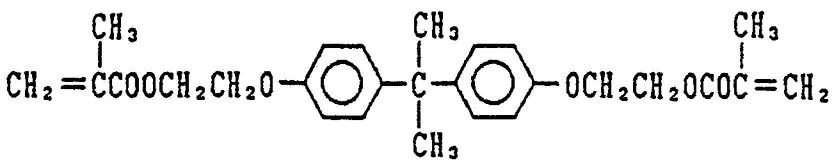

An object of the invention is to provide a novel dental material which exhibits superior characteristics to those of polymers of the above compounds.

The above and other objects of the invention will become apparent from the following description.

The present invention provides a high-molecular-weight dental material comprising a copolymer which contains at least 10% by weight of at least one structural unit derived from the following monomers

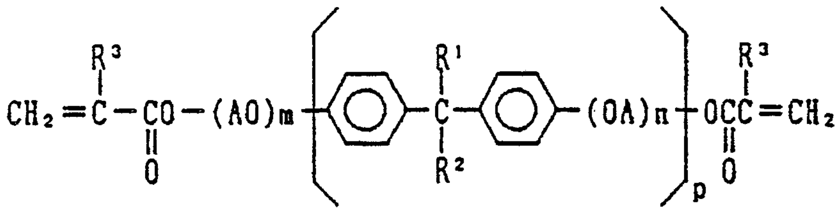

(1)

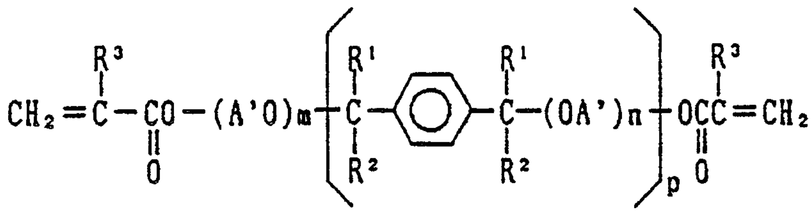

(2)

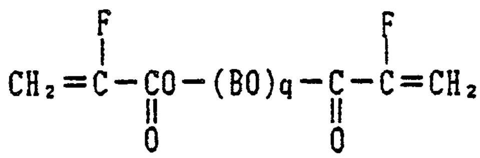

(3)

wherein $R^1$ and $R^2$ are each H, $CH_3$ or $CF_3$, $R^3$ is H, $CH_3$ or F, A is $-CH_2-$, $-CH_2CH_2-$, $-CH_2CH(CH_3)-$, $-CH(OH)-$, $-CH_2CH(OH)CH_2-$, $-CH(OCOCH_3)-$ or $-CH_2CH(OCOCH_3)CH_2-$, $A'$ is the same group as A or $-CH_2CH_2CH_2-$, B is alkylene group having 2 to 10 carbon atoms, m and n are each zero or a number of 1 to 5, p is a number of 1 to 3, q is a number of 1 to 5, provided that at least one of $R^1$ and $R^2$ if $CF_3$ when $R^3$ is H or $CH_3$.

In the above, A represents $-CH_2-$, $-CH_2CH_2-$, $-CH_2CH(CH_3)-$, $-CH(OH)-$, $-CH_2CH(OH)CH_2-$, $-CH(OCOCH_3)-$ or $-CH_2CH(OCOCH_3)CH_2-$,
$A'$ represents the same group as A or $-CH_2CH_2CH_2-$, and B is an alkylene group having 2 to 10, preferably 2 to 6,and particularly 2 to 4 carbon atoms which may be branched or straight-chain.

The dental material of the present invention can be used in various fields, for example, in crown restoration material, adhesive material, artificial crown material and denture base material.

The polymer of the present invention can be prepared by copolymerizing at least 10%, preferably at least 20%,and most preferred at least 30% by weight of at least one monomer of the above formulae (1) to (3), and up to 90% by weight of other monomers.

The preferred examples of other monomers are acrylic acid, methacrylic acid and esters thereof. The esters are preferably alkyl ($C_1$~20) esters, and polyethylene glycol, trimethylolpropane and like polyalcohol esters such as polyethylene glycol diacrylate and trimethylolpropane triacrylate.

It is possible to use other monomers in an amount which does not deteriorate and characteristics of the present dental material.

The present copolymer can be prepared by polymerising the above monomers by a conventional method used in a polymerization of an ethylenically unsaturated monomer. For example, the present copolymer is obtained preferably by a bulk polymerization in which molding is easily conducted, or by powder-liquid polymerization which is often used in dentistry. Further, the copolymer can by prepared by a photopolymerization, irradiating with ultraviolet or visible light, or by a suspension polymerization. It is preferable to use a polymerization initiator in the polymerization with heating.

As the polymerization initiator is usually used a compound which decomposes at around the room temperature and produces a radical. The polymerization initiator includes tri-n-butylborane and like alkylated metals which react with oxygen to produce a radical, peroxides (benzoyl peroxide, acetyl peroxide. lauroyl peroxide, cumene hydroperoxide, methyl ethyl ketone peroxide, t-butyl peroxybenzoate, etc.) and a combination of the above peroxides and accelerators (tertiary amine, cobalt salt of naphthenic acid or octenoic acid, transition metal ion, p-toluenesulfonic acid, amine salt of sulfinic acid, etc.). Each of the above polymerization initiators and accelerators is used preferably in an amount of about 0.1 to 2.5 parts by weight per 100 parts by weight of the monomer.

A photosensitizer is usually used when the monomer is polymerized by irradiation with ultraviolet or visible light. As the photosensitizer, benzophenone, nitrofluorene, 5-nitroacenaphthene or the like is used when ultraviolet light is employed, whereas camphorquinone or the like is used when visible light is

employed.
camphorquinone or the like is used when visible ray is irradiated.

To the present dental material, it is possible to add an additive which is used in a plastic dental material, such as silica, alumina and silicon nitride.

The present dental material has an equivalent or superior mechanical strength to those prepared by use of the conventional Bis-DMA, 3G or Bis-GMA, and also is excellent particularly in dimensional stability and non-water-absorbency, hence excellent in durability.

In the present invention, at least one of the monomers of the formulae (1) to (3) may be used in the form of a polymer. Alternatively the monomer is added to a dental composition and then polymerized in a process of preparing a dental material.

The present invention will be explained by the following examples. The parts are all by weight.

## Example 1

Into a glass tube were placed 29 parts of 1,1,1,3,3,3-hexafluoro-2,2-bis(4-methacryloxyphenyl)propane (Bis F-DMA) of the formula

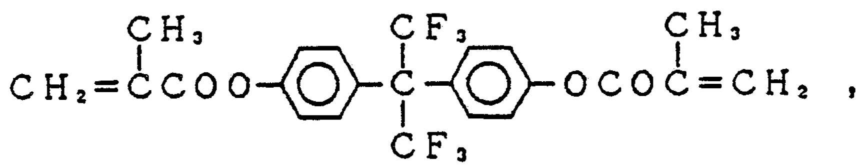

71 parts of methyl methacrylate (MMA) and 0.5 part of benzoyl peroxide. The glass tube was sealed and the content was bulk polymerized at 55 °C for 24 hours and further at 100 °C for 15 hours to obtain Copolymer A.

## Example 2

A polymerization was conducted in the same manner as in Example 1 except that 2,2-bis[4-(α-fluoroacryloxy)phenyl]propane (Bis-DFA) of the formula

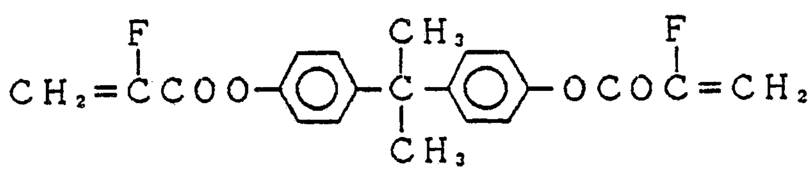

was used in place of Bis F-DMA to obtain Copolymer B.

## Example 3

A polymerization was conducted in the same manner as in Example 1 except that 1,1,1,3,3,3-hexafluoro-2,2-bis[4-(α-fluoroacryloxy)phenyl]propane (Bis F-DFA) of the formula

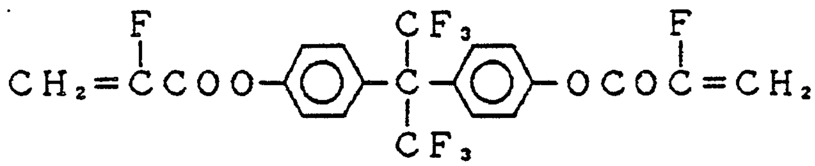

was used in place of Bis F-DMA to obtain Copolymer C.

## Example 4

A polymerization was conducted in the same manner as in Example 1 except that 38 parts of 1,4-bis-(1,1,1,3,3,3-hexafluoro-2-methacryloxypropyl)benzene (FB-DMA) of the formula

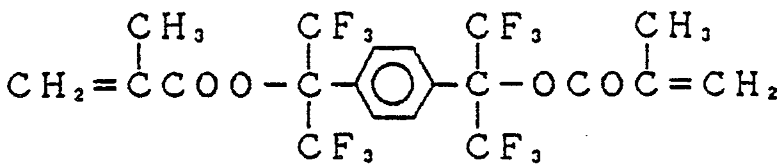 ,

MMA (62 parts) and 0.5 part of benzoyl peroxide were used to prepare Copolymer D.

## Example 5

A polymerization was conducted in the same manner as in Example 4 except that 1,4-bis(1,1,1,3,3,3-hexafluoro-2-fluoroacryloxypropyl)benzene [a mixture of meta compound and para compound (molar ratio = 4/1), FB-DFA] was used in place of FB-DMA to obtain Copolymer E.

## Example 6

A polymerization was conducted in the same manner as in Example 1 except that 41 parts of 1,1,1,3,3,3-hexafluoro-2,2-bis[4-(3-methacryloxy-2-hydroxypropoxy)phenyl]propane (Bis F-GMA) of the formula

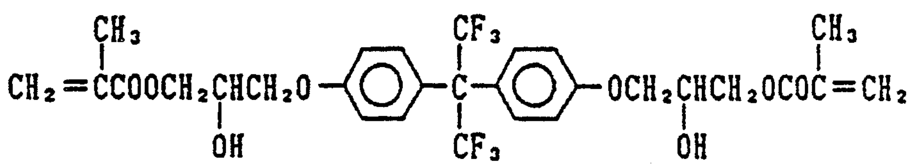 ,

59 parts of MMA and 0.5 parts of benzoyl peroxide were employed to obtain copolymer F.

## Example 7

A polymerization was conducted in the same manner as in Example 1 except that 44 parts of

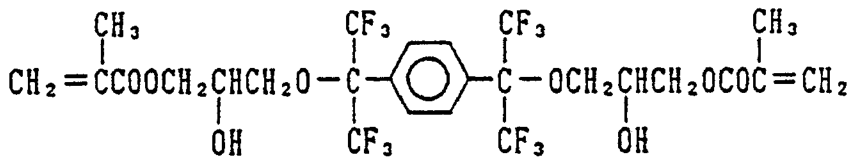

(FB-GMA), MMA (56 parts) and 0.5 part of benzoyl peroxide were used to obtain Copolymer G.

## Comparison Example 1

A polymerization was conducted in the same manner as in Example 1 except that 2,2-bis(4-methacryloxypheny)propane (Bis-DMA) of the formula

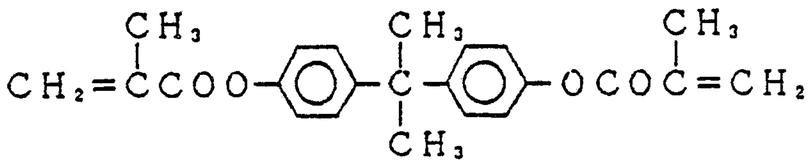

was used in place of Bis F-DMA to prepare Copolymer H.

## Comparison Example 2

A polymerization was conducted in the same manner as in Example 1 except that 24 parts of triethylene glycol dimethacrylate (3G), MMA (76 parts) and 0.5 part of benzoyl peroxide were used to prepare Copolymer I.

## Comparison Example 3

A polymerization was conducted in the same manner as in Example 1 except that 36 parts of 2,2-bis[4-(3-methacryloxy-2-hydroxypropoxy)phenyl]propane (Bis-GMA), MMA (64 parts) and 0.5 part of benzoyl peroxide were used to obtain Copolymer J.

## Test Example 1

The copolymers of Examples and Comparison Examples were checked for mechanical properties. The results are given in Table 1.

Table 1

| | copolymer | compressive strength (kgf/cm²) | indirect tensile strength (kgf/cm²) | bending strength (kgf/cm²) | bending elasticity (×10²kgf/cm²) | abrasion volume (mm³) |
|---|---|---|---|---|---|---|
| Ex. 1 | A | 1550 | 550(a) | 3500 | 530 | 6.0 |
| Ex. 2 | B | 1680 | 610(a) | 3750 | 760 | 4.9 |
| Ex. 3 | C | 1680 | 540(a) | 3250 | 530 | 5.1 |
| Ex. 4 | D | 1280 | 550(b) | 3500 | 520 | 10.3 |
| Ex. 5 | E | 1330 | 520(b) | 3500 | 535 | 8.7 |
| Ex. 6 | F | 1380 | 450(b) | 3200 | 550 | 8.4 |
| Ex. 7 | G | 1340 | 470(b) | 3100 | 580 | 10.4 |
| Com.Ex. 1 | H | 1550 | 500(a) | 3500 | 550 | 5.8 |
| Com.Ex. 2 | I | 1200 | 525(b) | 3250 | 430 | 6.8 |
| Com.Ex. 3 | J | 1340 | 510(b) | 3600 | 535 | 7.9 |

Test method

(1) Compressive strength test

As a sample was used a copolymer of 4 mm in diameter and of 8 mm in height which was immersed in water at 37° C for 24 hours. The sample was tested by No. 9 method of American Dental Association (ADA) at a crosshead speed of 2.5mm/min. with use of an autograph IS-500 of Shimadzu Seisakusho.

(2) Indirect tensile strength test

As a sample was used a copolymer of 6 mm in diameter and of 5 mm in height which was immersed in water at 37° C for 24 hours. The sample was tested by Diametral method at the same crosshead speed as above. In the Table, (a) shows proportional breaking and (b) shows breaking point.

(3) Bending strength and bending elasticity test

As a sample was used a copolymer of 4 mm in diameter and of 25mm in height which was immersed in water at 37° C for 50 hours. The three samples were tested by bending test method (distance between supports : 20mm) at a crosshead speed of 1 mm/min.

(4) Abrasion test with a toothbrush

As a sample was used a copolymer of 10Ø × 2 mm. The sample was checked for weight loss by use of an abrasion test machine K172 of Tokyo Giken Co., Ltd., at 5 cm of one stroke, load of 132g/sample and 30000 strokes. Abrasion volume was calculated from the weight loss and specific gravity of the sample.

Test Example 2

Water absorption amount (mg/cm²) was measured with use of copolymer samples (10Ø × 1 mm) of Examples 1 to 5 and Comparison Example 1 after immersed in water at 37° C for 7 days according to ADA standard. The results are shown in Table 2.

Table 2

| | water absorption amount ($mg/cm^2$) | | water absorption amount ($mg/cm^2$) |
|---|---|---|---|
| Ex. 1 | 0.80 | Ex. 4 | 0.74 |
| 2 | 0.76 | 5 | 0.57 |
| 3 | 0.70 | Com.Ex. 1 | 1.00 |

## Test Example 3

Water absorption amount was measured with respect to copolymers of Examples 6 and 7 and Comparison Examples 2 and 3 in the same manner as in Test Example 2. The results are given in Table 3.

Table 3

| | water absorption amount ($mg/cm^2$) | | water absorption amount ($mg/cm^2$) |
|---|---|---|---|
| Ex. 6 | 0.99 | Com. Ex. 2 | 1.38 |
| 7 | 1.07 | 3 | 1.60 |

## Example 8

Into a cylindrical vessel, 4 mm in diameter and 6 mm in depth, were placed a mixture of 2 ml of a composition and 3 g of filler, the composition being composed of Bis-DFA(2g), 3GF(5 g), CQ(0.07g) and DEAEM(0.156g), and the filler being Aerosil OX50 treated with 3-MPS (5 wt%). The vessel was capped with a celluloid plate and the content was irradiated with a light having a wavelength of 494nm for 60 seconds to obtain a photocured product having a depth of 5.4mm.

In the above,

3GF : Triethylene glycol di(α-fluoromethacrylate)

CQ : Camphorquinone

DEAEM: 2-Diethylaminoethyl methacrylic acid

## Comparison Example 4

A photocured product having a depth of 2.1mm was obtained in the same manner as in Example 8, except that Bis-DMA and 3G were used respectively in place of Bis-DFA and 3GF.

It is apparent from the above the cured product of Example 8 has about 2.6 times dimensional stability (small in shrinkage) than that of Comparison Example 4.

## Example 9

On a glass plate of 6 × 6 × 3 mm was placed about 0.01 g of a mixture of Example 8. Said mixture was irradiated from 2 mm-distance with light having a wavelength of 494nm for 30 seconds to obtain a photocured product. The product had a surface hardness (Vickers hardness, Hv) of 13.5.

Comparison Example 5

A photocured product having a surface hardness (Hv) of 2.0 was obtained in the same manner as in Example 9, except that a mixture of Comparison Example 4 was used.

It is apparent from the above that the cured product of Example 9 has an about 6.8 times higher surface hardness than that of Comparison Example 5.

## Claims

1. A dental material comprising a copolymer which contains at least 10% by weight of at least one structural unit derived from the following monomers

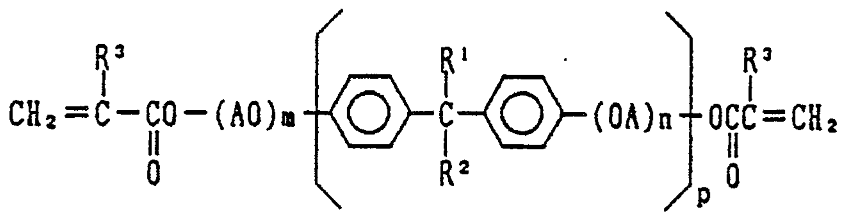

(1)

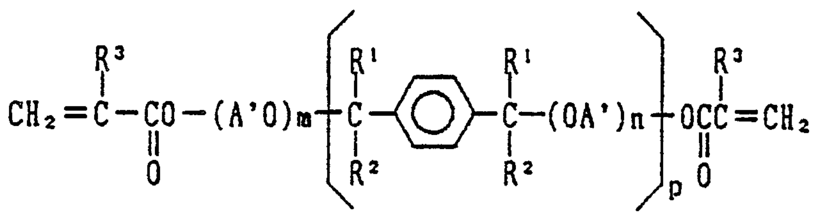

(2)

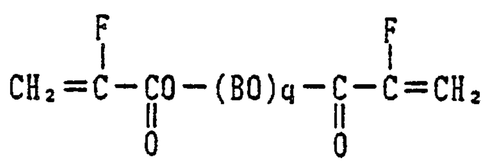

(3)

wherein $R^1$ and $R^2$ are each H, $CH_3$ or $CF_3$, $R^3$ is H, $CH_3$ or F, A is $-CH_2-$, $-CH_2CH_2-$, $-CH_2CH(CH_3)-$, $-CH(OH)-$, $-CH_2CH(OH)CH_2-$, $-CH(OCOCH_3)-$ or $-CH_2CH(OCOCH_3)CH_2-$, A′ is the same group as A or $-CH_2CH_2CH_2-$, B is alkylene group having 2 to 10 carbon atoms, m and n are each zero or a number of 1 to 5, p is a number of 1 to 3, q is a number of 1 to 5, provided that at least one of $R^1$ and $R^2$ is $CF_3$ when $R^3$ is H or $CH_3$.

2. A dental material as defined in claim 1 wherein the monomer is copolymerized conjointly with acrylic acid, methacrylic acid or an ester thereof.

3. A dental material as defined in claim 2 wherein the monomer is copolymerized conjointly with methyl methacrylate.

European Patent Office

# EUROPEAN SEARCH REPORT

Application Number

EP 88 11 4076

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 210 850 (MONTEDISON)<br>* Claims; example 1; column 7, lines 35-50 *<br>--- | 1-3 | A 61 K 6/08 |
| X | US-A-4 616 073 (J. ANTONUCCI)<br>* Column 2, line 57 - column 4, line 35; column 10, lines 3-5; claims *<br>--- | 1-3 | |
| X | EP-A-0 168 032 (DAIKIN)<br>* Claims; page 1, lines 5-12; page 2, line 20 - page 4, line 25; page 14, line 23 - page 15, line 13; page 16, lines 5-6 *<br>--- | 1-3 | |
| A | EP-A-0 211 408 (DAIKIN)<br>* Claims; page 5, line 23 - page 6, line 7 *<br>----- | 1-3 | |
| | | | TECHNICAL FIELDS SEARCHED (Int. Cl.4) |
| | | | A 61 K<br>C 08 F |

The present search report has been drawn up for all claims

| Place of search | Date of completion of the search | Examiner |
|---|---|---|
| THE HAGUE | 07-12-1988 | COUSINS-VAN STEEN G.I.L. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)